# EUROPEAN PATENT SPECIFICATION

(11) **EP 3 985 019 B1**
(45) Date of publication and mention of the grant of the patent: **18.09.2024**
(21) Application number: 20932054.8
(22) Date of filing: 09.06.2020
(51) Int. Cl.: C07K 14/78

(54) **RECOMBINANT HUMAN COLLAGEN AND CONSTRUCTION METHOD THEREFOR**
REKOMBINANTES MENSCHLICHES KOLLAGEN UND VERFAHREN ZU DESSEN HERSTELLUNG
COLLAGÈNE HUMAIN RECOMBINANT ET SON PROCÉDÉ DE CONSTRUCTION

(30) Priority: 24.04.2020 CN 202010331037
(43) Date of publication of application: 20.04.2022
(73) Proprietor: Nantong University, Nantong, Jiangsu 226019 (CN)
(72) Inventor: GU, Xiaosong, Nantong, Jiangsu 226019 (CN); QIAN, Tianmei, Nantong, Jiangsu 226019 (CN); WANG, Hongkui, Nantong, Jiangsu 226019 (CN); SUN, Hualin, Nantong, Jiangsu 226019 (CN); GU, Yun, Nantong, Jiangsu 226019 (CN)
(74) Representative: Grünecker Patent- und Rechtsanwälte PartG mbB
(86) International application number: PCT/CN2020/095014
(87) International publication number: WO 2021/212627

(56) References cited:
- WO-A1-2009/053985
- WO-A1-2016/090892
- CN-A- 102 925 451
- CN-A- 110 194 795
- CN-A- 111 004 319
- US-A- 5 710 252
- US-B1- 6 992 172
- TAKEOKA YOSHIKI ET AL: "Reduced nucleotomy-induced intervertebral disc disruption through spontaneous spheroid formation by the Low Adhesive Scaffold Collagen (LASCol)", BIOMATERIALS, vol. 235, 1 March 2020 (2020-03-01), AMSTERDAM, NL, pages 119781, XP055970001, ISSN: 0142-9612, DOI: 10.1016/j.biomaterials.2020.119781
- YAN WANG ET AL: "Nanoparticle-Based Delivery System for Application of siRNA In Vivo", CURRENT DRUG METABOLISM, vol. 11, no. 2, 1 February 2010 (2010-02-01), US, pages 182 - 196, XP055681520, ISSN: 1389-2002, DOI: 10.2174/138920010791110863
- HUBMACHER DIRK, SABATIER LAETITIA, ANNIS DOUGLAS S., MOSHER DEANE F., REINHARDT DIETER P.: "Homocysteine Modifies Structural and Functional Properties of Fibronectin and Interferes with the Fibronectin–Fibrillin-1 Interaction", BIOCHEMISTRY, vol. 50, no. 23, 14 June 2011 (2011-06-14), pages 5322 - 5332, XP055859959, ISSN: 0006-2960, DOI: 10.1021/bi200183z
- KULKE MARTIN, GEIST NORMAN, FRIEDRICHS WENKE, LANGEL WALTER: "Molecular dynamics simulations on networks of heparin and collagen : MD on Networks of Heparin and Collagen", PROTEINS: STRUCTURE, FUNCTION, AND BIOINFORMATICS, vol. 85, no. 6, 1 June 2017 (2017-06-01), US , pages 1119 - 1130, XP055859962, ISSN: 0887-3585, DOI: 10.1002/prot.25277
- WAN, TAO ET AL.: "The Multi-Parameter Prediction of Potential Protein Antigenic Determinants", CHINESE JOURNAL OF IMMUNOLOGY, vol. 13, no. 6, 31 December 1997 (1997-12-31), pages 329 - 333, XP055859967

## Description

### Technical Field

The present invention belongs to the field of biotechnologies, and specifically to a recombinant human collagen and a method for building same.

### Background

Collagen is the most abundant protein in mammals, accounts for about 30% of the total protein content, is widely found in animal skin, cartilage, blood vessels, and other tissues and organs, and participates in the growth, development and cellular differentiation of organisms, adhesion, binding of antigen and antibody, and other important life processes. So far, 28 different types of collagen have been identified in vertebrates and higher invertebrates. Among these types of collagen, collagen type I is the most abundant collagen in vertebrates, and is combined with other molecules in different proportions to form various tissue scaffolds such as basement membrane, ligaments, tendons, skin, and blood vessels, to provide such tissue scaffolds with high mechanical strength.

The most typical structure of collagen is a fibrous protein formed by the twisting of peptide chains with a triple-helical structure. The ordered aggregation of the triple-helical molecules makes the molecular structure very stable and provides fibers with high ductility. In addition, the triple-helical structure allows for better mechanical strength of collagen. The amino acid arrangement of the primary structure of collagen presents a periodic pattern "Gly-X-Y (X and Y are any amino acids other than glycine)".

Collagen is widely applied to food industry, pharmaceutical industry, cosmetic industry, among other industries because of its excellent biological properties such as low immunogenicity, good biocompatibility, and biodegradability. In recent years, collagen has been widely used in medicine in the form of membranes, sponges, injections, plugs, and the like for cosmetic, orthopedic, burn, trauma, hard tissue repair, and the like. However, natural collagen is insoluble in water. Moreover, the nature of collagen extracted from animals is not homogeneous, making further treatment very difficult. At present, for the production of collagen, collagen is mainly obtained by using acid, alkaline, and various enzymatic methods from connective tissues of animals such as pigskin, cowhide or fish skin. However, animal-derived collagen has many virus risks, and causes xenograft rejection when acting on human body. Next, collagen synthesized by chemical methods cannot avoid the loss of biological activity. In addition, the techniques are relatively complex, and extraction methods cause heavy pollution and have high costs. As a result, the application of collagen to many fields such as the medicine field is greatly restricted.

With the rapid development of modern molecular biology, people are beginning to turn their attention to the use of genetic engineering techniques, and produce recombinant human collagens by using various host cells from insects, transgenic mice, E. coli, and the like. For example, Fan Dai-di et al. of Northwest University applied high-density fermentation culture of E. coli to produce human-like collagens. However, the expression level of protein is less than 30%. In addition, the bacterial expression system has biosafety issues such as endotoxins and heat sources, and a protein produced by expression is often present in the form of inclusion bodies. As a result, it is relatively difficult to purify the protein, and the product is impure and not easy to be clinically applied. Therefore, nowadays, more and more scholars have started to cultivate recombinant human collagens by fermentation using engineered Yeast Pichia Pastoris. Along with advantages such as no heat source and extracellular secretion of products, there are many deficiencies such as a long fermentation cycle, low production efficiency, and low purity. Therefore, in the field of technologies for preparing recombinant human collagens, there is an urgent need to develop a method with high purity, high safety, a high yield, a short cycle, high hydrophilicity, and easy mass production. In addition, for better clinical application and to avoid rejection in human body, it is necessary to develop a low-immunogenic recombinant human collagen.

### Summary

To overcome the deficiencies in the prior art, a technical problem to be resolved by the present invention is to provide a recombinant human collagen and a method for building same. The recombinant human collagen has a short peptide segment can be directly synthesized and is suitable for large-scale preparation, operations are simple, water solubility is high, and no antigenic determinant is found.

The technical solutions used in the present invention to resolve the foregoing technical problem are as follows:
According to a first aspect, a recombinant human collagen is provided. The amino acid sequence of the collagen is SEQ ID NO. 1 and is as follows:
GPAGARGNDGATGAAGPPGPTGPAGPPGFP.

Also disclosed, but not part of the invention, the recombinant human collagen has a single-chain single-helix structure, presents a sequence feature of the collagen"Gly-X-Y"(X and Y are any amino acids other than glycine)", has a full length of 30 amino acids, and is a human collagen type I peptide segment.

The recombinant human collagen has a molecular mass of 2526.71Da and a theoretical isoelectric point of 5.84.

The recombinant human collagen does not have a signal peptide and a transmembrane domain and is soluble in water.

No antigenic determinant is found in analysis of the recombinant human collagen by using an epitope prediction tool Predicted Antigenic Peptides.

It is found through online analysis by using SOPMA that the recombinant human collagen is most likely to form an irregular winding coil (randomcoil) structure.

The recombinant human collagen is a novel protein, and no subcellular localization is found.

The present invention further provides a method for building a recombinant human collagen, including:
step (1): acquiring a sequence of a human collagen type I from an NCBI database;
step (2): analyzing an antigenic determinant of the human collagen type I by using an online epitope prediction tool Predicted Antigenic Peptides, and performing screening to obtain a low antigenic determinant region; and
step (3): selecting a low antigenic determinant region from the selected low antigenic determinant region by using BitGene online antigenic determinant prediction (in combination with five algorithms: antigenicity, hydrophilicity, flexibility, surface accessibility, and β turn prediction), and determining the amino acid sequence of a recombinant human collagen that needs to be synthesized.

Further, the method further includes step (4):analyzingthe determined recombinant human collagen that needs to be synthesized by using the epitope prediction tool Predicted Antigenic Peptides to determine the immunogenicity of the recombinant human collagen.

The acquiring a sequence of a human collagen type I from an NCBI database in step (1) is as follows:
searching "recombinant human collagen"in the NCBI database Gene, and downloading the sequence of the human collagen type I of NP_000079.2, a length of the sequence being 1464 amino acids.

A process of obtaining the low antigenic determinant region in the foregoing step (2) is as follows:
analyzing the foregoing human collagen type I with a length of 1464 amino acids by using the online epitope prediction tool Predicted Antigenic Peptides (URL: http://imed.med.ucm.es/Tools/antigenic.pl) to determine the antigenic determinant of the human collagen type I, to select a low antigenic determinant region of 171 amino acids at the position 230 to the position 400.

A process of determining the amino acid sequence of the recombinant human-derived collagen that needs to be synthesized in the foregoing step (3) is as follows:
analyzing the human collagen with a length of the foregoing selected 171 amino acids by using five standard methods of epitope prediction: BitGene online Kolaskar & Tongaonkar Antigenicity, Parker Hydrophilicity, Karplus & Schulz Flexibility, Emini Surface Accessibility, and Chou & Fasman Beta-Turn, to determine an antigenic determinant of the low antigenic determinant region; and
selecting a low antigenic determinant, high hydrophilicity region, and preferentially selecting, in combination with a sequence feature of the collagen, that is, a presented arrangement pattern "Gly-X-Y (X and Y are any amino acids other than glycine)", a sequence of a low-immunogenic, hydrophilic recombinant human collagen that needs to be prepared.

The recombinant human collagen in the present invention has a short peptide segment, can be directly synthesized, has a high yield, a short cycle, high purity, and no virus risk, and is soluble in water.

Further, a recombinant DNA sequence for preparing a recombinant human collagen is further provided as follows:

The recombinant human collagen and the method for building same provided in the present invention have the following advantages as compared with the prior art:
1. The recombinant human collagen in the present invention has a short peptide segment with a length of only 30 amino acids and can be directly synthesized by a company. Prokaryotic or eukaryotic expression is not required. Operations are simple. It is easy to obtain a large amount of protein. There is no virus risk. The purity found through high performance liquid chromatography detection is up to 95%.
2. The recombinant human collagen in the present invention has low immunogenicity and is soluble in water. The amino acid composition of the recombinant human collagen is the same as that of a corresponding part of the amino acid sequence of a natural collagen. The collagen does not elicit immunological rejection when applied to human body and can be widely used clinically.

### Brief Description of the Drawings

FIG. 1 is an analysis chart of a signal peptide of a recombinant human collagen according to an embodiment of the present invention;
FIG. 2 is an analysis chart of a transmembrane domain of a recombinant human collagen according to an embodiment of the present invention;
FIG. 3 is an analysis chart of hydrophilicity of a recombinant human collagen according to an embodiment of the present invention;
FIG. 4 is an analysis chart of the structure of a recombinant human collagen according to an embodiment of the present invention;
FIG. 5 is a predicted chart of an antigenic determinant of a recombinant human collagen according to an embodiment of the present invention;
FIG. 6 is a cell localization diagram of a recombinant human collagen according to an embodiment of the present invention; and
FIG. 7 is a high performance liquid chromatograph of a recombinant human collagen according to an embodiment of the present invention.

### Detailed Description of Embodiments

The present invention is further described below in detail with reference to the accompanying drawings and embodiments.

### Embodiment 1

A method for building a recombinant human collagen includes:
(1) acquiring a sequence of a human collagen type I from an NCBI database;
(2) analyzing an antigenic determinant of the human collagen type I by using an online epitope prediction tool Predicted Antigenic Peptides, and performing screening to obtain a low antigenic determinant region;
(3) selecting a low antigenic determinant region from the selected region by using BitGene online antigenic determinant prediction (in combination with five algorithms: antigenicity, hydrophilicity, flexibility, surface accessibility, and β turn prediction), and determining a recombinant human collagen that needs to be synthesized; and
(4) analyzing the determined recombinant human collagen that needs to be synthesized by using the epitope prediction tool Predicted Antigenic Peptides to determine the immunogenicity of the recombinant human collagen.

The acquiring a sequence of a human collagen type I from an NCBI database in step (1) is as follows:
searching "recombinant human collagen"in the NCBI database Gene, and downloading the sequence of the human collagen type **I** of NP_000079.2, a length of the sequence being 1464 amino acids.

A process of obtaining the low antigenic determinant region in the foregoing step (2) is as follows:
analyzing the foregoing human collagen type I with a length of 1464 amino acids by using the online epitope prediction tool Predicted Antigenic Peptides (URL: http://imed.med.ucm.es/Tools/antigenic.p1) to determine the antigenic determinant of the human collagen type I, to select a low antigenic determinant region of 171 amino acids at the position 230 to the position 400.

A process of determining the amino acid sequence of the recombinant human-derived collagen that needs to be synthesized in the foregoing step (3) is as follows:
analyzing the human collagen with a length of the foregoing selected 171 amino acids by using five standard methods of epitope prediction: BitGene online Kolaskar & Tongaonkar Antigenicity, Parker Hydrophilicity, Karplus & Schulz Flexibility, Emini Surface Accessibility, and Chou & Fasman Beta-Turn, to determine an antigenic determinant of the low antigenic determinant region; and
selecting a low antigenic determinant, high hydrophilicity region, and preferentially selecting, in combination with a sequence feature of the collagen, that is, a presented arrangement pattern "Gly-X-Y (X and Y are any amino acids other than glycine)", the amino acid sequence of a low-immunogenic, hydrophilic recombinant human collagen that needs to be prepared (SEQ ID NO. 1)as follows: GPAGARGNDGATGAAGPPGPTGPAGPPGFP.

### Embodiment 2: Method for synthesizing a recombinant human collagen

A method for synthesizing a recombinant human collagen includes the following steps:
1. swelling solid phase resin with dichloromethane for 15 minutes, drawing off the solution, releasing fluorenylmethoxycarbonyl with a 20% piperidine/dimethylformamide solution for 15 minutes, performing washing 9 times with dimethylformamide, weighing protected amino acids, O-benzotriazole-tetramethylurea hexafluorophosphate, 1-hydroxybenzotriazole, and N,N-diisopropylethylamine for 40 minutes of condensation reaction, washing the resin 6 times with dimethylformamide after the reaction has been performed for 40 minutes, and taking a small amount of resin for reaction monitoring with a ninhydrin solution, where the reaction is complete when the solution is colorless;
2. releasing fluorenylmethoxycarbonyl with a 20% piperidine/dimethylformamide solution for 15 minutes, performing washing 9 times with dimethylformamide, weighing protected amino acids, O-benzotriazole-tetramethylurea hexafluorophosphate, 1-hydroxybenzotriazole, and N,N-diisopropylethylamine for 40 minutes of condensation reaction, washing the resin 6 times with dimethylformamide after the reaction has been performed for 40 minutes, taking a small amount of resin for reaction monitoring with a ninhydrin solution, where the reaction is complete when the solution turns colorless, and this process is repeated until the reaction reaches the last amino acid; and
3. performing acid lysis on resin peptides with 95% TFA/water for 2 hours, performing filtering to remove resin, adding a stock solution to ether to precipitate polypeptides, and centrifuging and precipitating the solution to obtain the polypeptides; and performing high performance liquid chromatography purification on the crude polypeptides, and freeze-drying the crude polypeptides to obtain pure polypeptides.

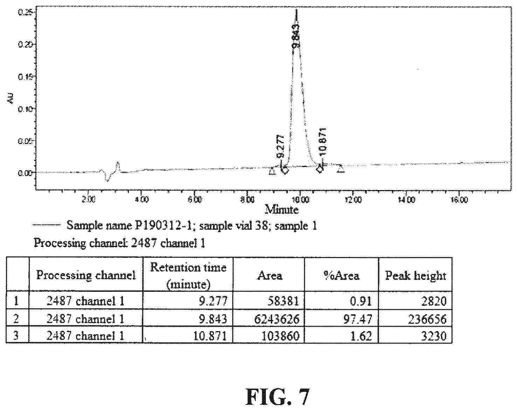

The purity of the synthesized recombinant human collagen found through high performance liquid chromatography detection is up to 95% (as shown in FIG. 7).

As observed with a microscope, the prepared recombinant human collagen has a random coil structure.

A water solubility test of the recombinant human collagen is as follows:
dissolving 32 mg of the recombinant human collagen in 8 ml of water directly without stirring, to form a clear solution with no color, no precipitation, and no floating matter. It indicates that the recombinant human collagen prepared in the present invention has adequate water solubility, and it is verified that the recombinant human collagen is a hydrophilic protein.

The recombinant human collagen synthesized by using the method has high purity, no virus risk, and a high expression level. The protein can be widely applied to related biomedical products, regenerative medicine products, tissue engineering and beauty products, health care products, cosmetic products, among others.

### Embodiment 3: Analysis of basic properties of a recombinant human collagen

A recombinant human collagen has a sequence (SEQ ID NO. 1) as follows:
GPAGARGNDGATGAAGPPGPTGPAGPPGFP.

The recombinant human collagen has a single-chain single-helix structure, presents a sequence feature of the collagen G-X-Y (X and Y are any amino acids other than glycine), has a full length of 30 amino acids, and is a human collagen type I peptide segment.

The recombinant human collagen has a molecular mass of 2526.71Da and a theoretical isoelectric point of 5.84.

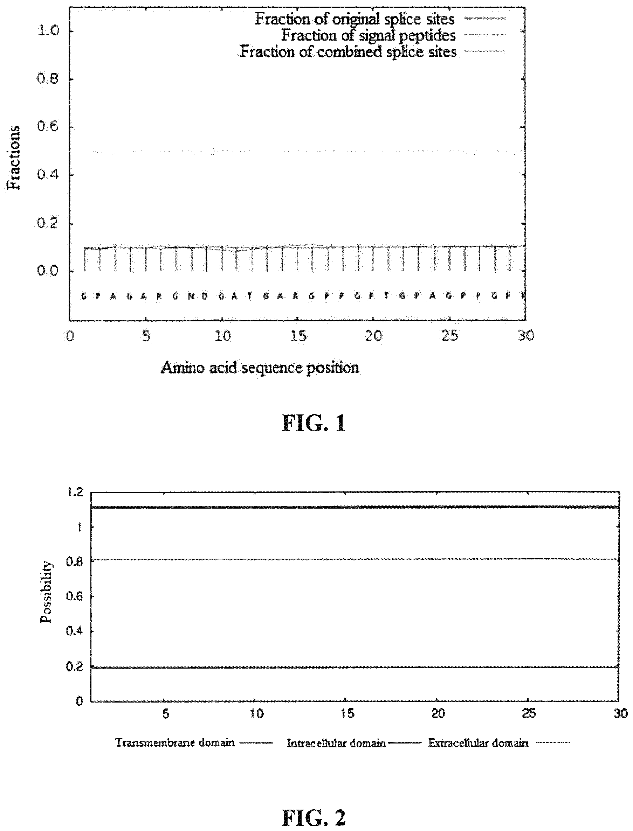

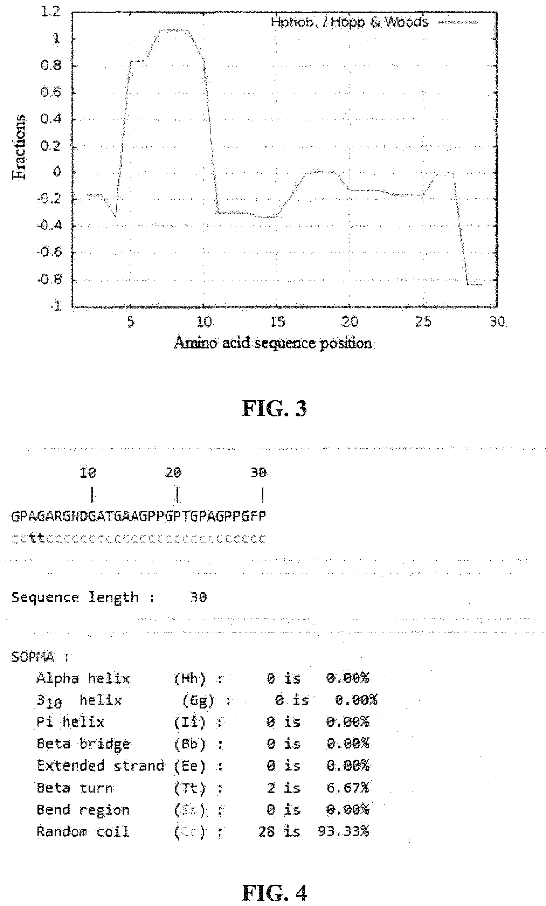

As shown in FIG. 1, the recombinant human collagen is analyzed by using a signal peptide prediction tool SignalP, to findthat the protein has no signal peptide. As shown in FIG. 2, the recombinant human collagen is analyzed by using TMHMM prediction to find that the protein does not have a transmembrane domain. As shown in FIG. 3, the recombinant human collagen is analyzed by using ExPASy to find that the protein is a hydrophilic protein. As shown in FIG. 4, online analysis SOPMA is performed on the recombinant human collagen to find that the protein is most likely to form an irregular winding coil (random coil) structure.

### Embodiment 4: Analysis of an antigenic determinant of a recombinant human collagen

A recombinant human collagen has a sequence (SEQ ID NO. 1) as follows:
GPAGARGNDGATGAAGPPGPTGPAGPPGFP.

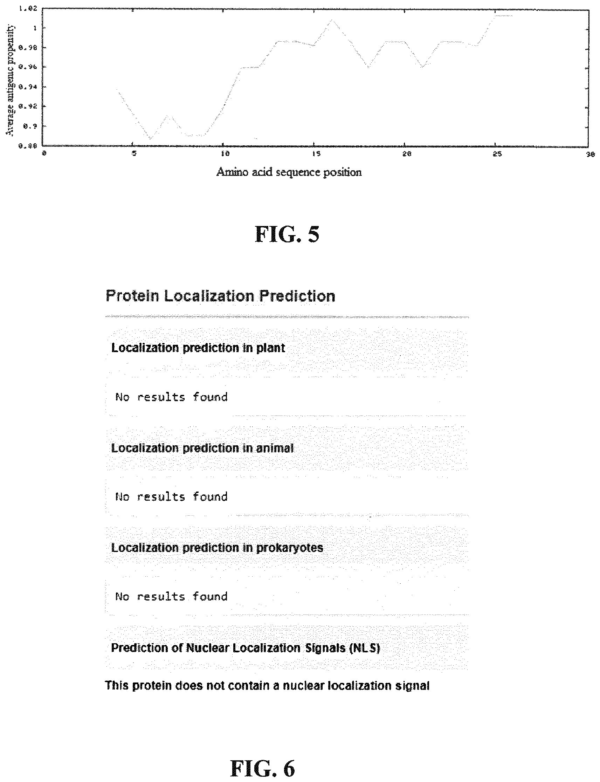

The protein is analyzed by using an online epitope prediction tool Predicted Antigenic Peptides (URL: http://imed.med.ucm.es/Tools/antigenic.pl) to determine an antigenic determinant of the protein. The average antigenicity of the protein is 0.9687. An antigenic determinant is not found (as shown in FIG. 5).

### Embodiment 5: Cell localization of a recombinant human collagen

A recombinant human collagen has a sequence (SEQ ID NO. 1) as follows:
GPAGARGNDGATGAAGPPGPTGPAGPPGFP

The protein is analyzed online by using PredictProtein (URL: https://www.predictprotein.org/home) to find that the protein is a novel protein. No subcellular localization is found (as shown in FIG. 6).

## Claims

1. A recombinant human collagen, wherein the amino acid sequence of the recombinant human collagen is SEQ ID NO. 1: GPAGARGNDGATGAAGPPGPTGPAGPPGFP.

2. The recombinant human collagen according to claim 1, wherein the recombinant human collagen has a molecular mass of 2526.71Da and a theoretical isoelectric point of 5.84.

3. The recombinant human collagen according to claim 1, wherein the recombinant human collagen does not have a signal peptide and a transmembrane domain and is a hydrophilic protein.

4. The recombinant human collagen according to claim 1, wherein the recombinant human collagen is free of an antigenic determinant and is a low-immunogenic protein.

5. The recombinant human collagen according to claim 1, wherein the recombinant human collagen is capable of forming a random coil structure.

6. A method for building a recombinant human collagen according to claim 1, comprising:
step (1): acquiring a sequence of a human collagen type I from an NCBI database;
step (2): analyzing an antigenic determinant of the human collagen type I by using an online epitope prediction tool Predicted Antigenic Peptides, and performing screening to select a low antigenic determinant region; and
step (3): selecting a low antigenic determinant, hydrophilic region from the selected low antigenic determinant region by using BitGene online antigenic determinant prediction and in combination with five algorithms: hydrophilicity, flexibility, surface accessibility, antigenicity, and β turn prediction, and determining, in combination with a sequence feature of the collagen, the amino acid sequence of a recombinant human collagen that needs to be synthesized.

7. The method for building a recombinant human collagen according to claim 6, wherein the human collagen type I has a sequence number of NP_000079.2 and has a length of 1464 amino acids.

8. The method for building a recombinant human collagen according to claim 7, wherein in step (2), the selected low antigenic determinant region is 171 amino acids at the position 230 to the position 400 in the human collagen type I.

9. The method for building a recombinant human collagen according to claim 8, wherein in step (2), a low antigenic determinant region of 171 amino acids is analyzed by using five standard methods of epitope prediction: BitGene online antigenicity, hydrophilicity, flexibility, surface accessibility, and β turn, to determine an antigenic determinant of the low antigenic determinant region; and
a low antigenic determinant, high hydrophilicity region is selected, and the amino acid sequence of the recombinant human-derived collagen that needs to be synthesized is determined in combination with the sequence feature of the collagen, that is, a presented arrangement pattern "Gly-X-Y, X and Y being any amino acids other than glycine".

## Patentansprüche

1. Rekombinantes humanes Kollagen, wobei die Aminosäuresequenz des rekombinanten humanen Kollagens SEQ ID NO. 1: GPAGARGNDGATGAAGPPGPTGPAGPPGFP ist.

2. Rekombinantes humanes Kollagen nach Anspruch 1, wobei das rekombinante humane Kollagen ein Molekulargewicht von 2526,71 Da und einen theoretischen isoelektrischen Punkt von 5,84 aufweist.

3. Rekombinantes humanes Kollagen nach Anspruch 1, wobei das rekombinante humane Kollagen kein Signalpeptid und keine Transmembrandomäne aufweist und ein hydrophiles Protein ist.

4. Rekombinantes humanes Kollagen nach Anspruch 1, wobei das rekombinante humane Kollagen frei von einer antigenen Determinante ist und ein Protein mit niedriger Immunogenität ist.

5. Rekombinantes humanes Kollagen nach Anspruch 1, wobei das rekombinante humane Kollagen dazu in der Lage ist, eine zufällige Spiralstruktur zu bilden.

6. Verfahren zum Erstellen eines rekombinanten humanen Kollagens nach Anspruch 1, umfassend:
Schritt (1): Erlangen einer Sequenz eines humanen Kollagentyps I aus einer NCBI-Datenbank;
Schritt (2): Analysieren einer antigenen Determinante des humanen Kollagentyps I unter Verwendung von antigenen Peptiden, die mit einem Online-Epitopvorhersagewerkzeug vorhergesagt wurden, und Durchführen eines Screenings, um eine Region mit geringer antigener Determinante zu wählen; und
Schritt (3): Auswählen einer hydrophilen Region mit geringer antigener Determinante aus der ausgewählten Region mit geringer antigener Determinante unter Verwendung von BitGene-Online-Vorhersage für antigene Determinanten und in Kombination mit fünf Algorithmen: Hydrophilie, Flexibilität, Zugänglichkeit der Oberfläche, Antigenizität und β-Tum-Vorhersage, und Bestimmen, in Kombination mit einem Sequenzmerkmal des Kollagens, der Aminosäuresequenz eines rekombinanten humanen Kollagens, das zu synthetisieren ist.

7. Verfahren zum Erstellen eines rekombinanten humanen Kollagens nach Anspruch 6, wobei der humane Kollagentyp I die Sequenznummer NP_000079.2 aufweist und eine Länge von 1464 Aminosäuren aufweist.

8. Verfahren zum Erstellen eines rekombinanten humanen Kollagens nach Anspruch 7, wobei in Schritt (2) die ausgewählte Region mit niedriger antigener Determinante 171 Aminosäuren an der Position 230 bis zu der Position 400 in dem humanen Kollagentyp I ist.

9. Verfahren zum Erstellen eines rekombinanten humanen Kollagens nach Anspruch 8, wobei in Schritt (2) eine Region mit niedriger antigener Determinante von 171 Aminosäuren unter Verwendung von fünf Standardverfahren der Epitopvorhersage analysiert wird: BitGene-Online-Antigenizität, Hydrophilie, Flexibilität, Zugänglichkeit der Oberfläche und β-Turn, um eine antigene Determinante der Region mit niedriger antigener Determinante zu bestimmen; und
eine Region mit niedriger antigener Determinante und hoher Hydrophilie ausgewählt wird und die Aminosäuresequenz des zu synthetisierenden rekombinanten humanen Kollagens in Kombination mit dem Sequenzmerkmal des Kollagens bestimmt wird, also einem präsentierten Anordnungsmuster "Gly-X-Y, X, wobei Y eine beliebige andere Aminosäure als Glycin ist".

## Revendications

1. Un collagène recombinant humain, dans lequel la séquence aminoacide du collagène recombinant humain est SEQ ID NO. 1 : GPAGARGNDGQTGAAGPPGPTGPAGPPGFP.

2. Un collagène recombinant humain selon la revendication 1, dans lequel le collagène recombinant humain est doté d'une masse moléculaire de 2526.71 DA et d'un point isoélectrique théorique de 5.84.

3. Un collagène recombinant humain selon la revendication 1, dans lequel le collagène recombinant humain n'est pas doté d'un peptide signal ni d'un domaine transmembranaire et est une protéine hydrophile.

4. Un collagène recombinant humain selon la revendication 1, dans lequel le collagène recombinant humain est exempt d'antigène déterminant et est une protéine à faible immunogénicité.

5. Un collagène recombinant humain selon la revendication 1, dans lequel le collagène recombinant humain est capable de former une structure de bobine aléatoire.

6. Un procédé pour construire un collagène recombinant humain selon la revendication 1, comprenant :
étape (1) : acquérir une séquence de collagène humain de type 1 depuis une base de données NCBI ;
étape (2) : analyser un déterminant antigénique du collagène humain de type 1 en utilisant l'outil de prédiction épitope en ligne Predicted Antigenic Peptides, et effectuer un filtrage pour sélectionner une région déterminante à faible antigénicité ; et
étape (3) : sélectionner une région déterminante à faible antigénicité hydrophile à partir de la région déterminante à faible antigénicité en utilisant la prédiction de déterminant antigénique en ligne BitGene et en combinaison avec cinq algorithmes : hydrophilie, flexibilité, accessibilité de surface, antigénicité, et prédiction de virage β, et déterminer, en combinaison avec une caractéristique de séquence du collagène, la séquence aminoacide d'un collagène recombinant humain qui doit être synthétisé.

7. Un procédé pour construire un collagène recombinant humain selon la revendication 6, dans lequel le collagène humain de type 1 est doté d'un numéro de séquence NP_000079.2 et est doté d'une longueur de 1464 aminoacides.

8. Un procédé pour construire un collagène recombinant humain selon la revendication 7, dans lequel à l'étape (2), la région déterminante à faible antigénicité sélectionnée est de 171 aminoacides de la position 230 à la position 400 dans le collagène humain de type I.

9. Un procédé pour construire un collagène recombinant humain selon la revendication 8, dans lequel à l'étape (2), la région déterminante à faible antigénicité de 171 aminoacides est analysée à l'aide de cinq procédés standards de prédiction épitope : antigénicité en ligne BitGene, hydrophilie, flexibilité, accessibilité de surface et virage β, pour déterminer un déterminant antigénique de la région déterminante à faible antigénicité ; et
une région déterminante à faible antigénicité, fortement hydrophile est sélectionnée, et la séquence aminoacide du collagène recombinant d'origine humaine qui doit être synthétisé est déterminée en combinaison avec la caractéristique de séquence du collagène, qui est un motif d'agencement présenté « Gly-X-Y, X et Y étant des aminoacides quelconques autres que la glycine. »


### Sequence Table

<110> Yaoshunze Biological Medicine (Nanjing) Co., Ltd.
<120>Recombinant human collagen and method for building same
<160>1
<170>SIPOSequenceListing 1.0
<210>1
   <211>30
   <212>PRT
   <213>Artificial Sequence
<400>1
   Gly Pro Ala Gly Ala Arg Gly Asn Asp Gly Ala Thr Gly Ala Ala Gly
   1 5 10
   15
   Pro Pro Gly Pro Thr Gly Pro Ala Gly Pro Pro Gly Phe Pro
   20 25 30